# EUROPEAN PATENT SPECIFICATION

(11) **EP 1 071 468 B1**
(45) Date of publication and mention of the grant of the patent: **14.06.2006**
(21) Application number: 99917558.1
(22) Date of filing: 15.04.1999
(51) Int. Cl.: A61K 47/48, A61K 38/39, A61K 39/395, A61K 38/19, A61K 31/405, C07K 19/00

(54) **ENHANCEMENT OF ANTIBODY-CYTOKINE FUSION PROTEIN MEDIATED IMMUNE RESPONSES BY CO-ADMINISTRATION WITH ANGIOGENESIS INHIBITOR**
STEIGERUNG DER ANTIKÖRPER-ZYTOKIN-FUSIONSPROTEINMEDIIERTEN IMMUNANTWORT DURCH MITVERABREICHUNG MIT EINEM ANGIOGENESEINHIBITOR
CO-ADMINISTRATION D'UN INHIBITEUR DE L'ANGIOGENESE POUR RENFORCER LA REPONSE IMMUNITAIRE A MEDIATION DE PROTEINE DE FUSION D'UNE CYTOKINE D'ANTICORPS

(30) Priority: 15.04.1998 US 81863 P
(43) Date of publication of application: 31.01.2001
(73) Proprietor: Lexigen Pharmaceuticals Corp., Lexington, MA 02421 (US)
(72) Inventor: GILLIES, Stephen, D., Carlisle, MA 01741 (US)
(74) Representative: Benz, Jürgen
(86) International application number: PCT/US1999/008335
(87) International publication number: WO 1999/052562

(56) References cited:
- EP-A- 0 706 799
- WO-A-92/02240
- WO-A-92/08495
- WO-A-97/15666
- WO-A-98/00127

### SEQUENCE LISTING

<110> Gillies, Stephen D
<120> Enhancement of Antibody-Cytokine Fusion Protein
   Mediated Immune Responses by Coadministration with
   Angiogenesis Inhibitor
<130> LEX-004PC
<140>
   <141>
<150> 60/081,863
   <151> 1998-04-14
<160> 20
<170> PatentIn Ver. 2.0
<210> 1
   <211> 24
   <212> DNA
   <213> Artificial Sequence
<220>
   <223> Description of Artificial Sequence:PCR Primer
<220>
   <221> misc_feature
   <222> (12)..(14)
   <223> Translation initiation codon
<400> 1
   tctagagcag catggcgccc ccgc 24
<210> 2
   <211> 24
   <212> DNA
   <213> Artificial Sequence
<220>
   <223> Description of Artificial Sequence:PCR Primer
<220>
   <221> misc_feature
   <222> (7).. (9)
   <223> Translation termination anticodon
<400> 2
   ctcgagttat gcattgagtt ccct 24
<210> 3
   <211> 35
   <212> DNA
   <213> Artificial Sequence
<220>
   <223> Description of Artificial Sequence:PCR Primer
<400> 3
   ccgtctcctc agccaaaaca acagccccat cggtc 35
<210> 4
   <211> 37
   <212> DNA
   <213> Artificial Sequence
<220>
   <223> Description of Artificial Sequence:PCR Primer
<400> 4
   ggggctgttg ttttggctga ggagacggtg actgacg 37
<210> 5
   <211> 25
   <212> DNA
   <213> Artificial Sequence
<220>
   <223> Description of Artificial Sequence:PCR Primer
<400> 5
   cttaagccag atccagttgg tgcag 25
<210> 6
   <211> 27
   <212> DNA
   <213> Artificial Sequence
<220>
   <223> Description of Artificial Sequence:PCR Primer
<400> 6
   cccggggtcc gggagaagct cttagtc 27
<210> 7
   <211> 30
   <212> DNA
   <213> Artificial Sequence
<220>
   <223> Description of Artificial Sequence:PCR Primer
<400> 7
   ggcccgggta aagcacccac ttcaagctcc 30
<210> 8
   <211> 25
   <212> DNA
   <213> Artificial Sequence
<220>
   <223> Description of Artificial Sequence:PCR Primer
<400> 8
   ccctcgagtt attgagggct tgttg 25
<210> 9
   <211> 32
   <212> DNA
   <213> Artificial Sequence
<220>
   <223> Description of Artificial Sequence:PCR Primer
<400> 9
   ggaaataaaa cgggctgatg ctgcaccaac tg 32
<210> 10
   <211> 34
   <212> DNA
   <213> Artificial Sequence
<220>
   <223> Description of Artificial Sequence:PCR Primer
<400> 10
   gcagcatcag cccgttttat ttccagcttg gtcc 34
<210> 11
   <211> 25
   <212> DNA
   <213> Artificial Sequence
<220>
   <223> Description of Artificial Sequence:PCR Primer
<400> 11
   cttaagcgag atcgtgctga cccag 25
<210> 12
   <211> 29
   <212> DNA
   <213> Artificial Sequence
<220>
   <223> Description of Artificial Sequence:PCR Primer
<400> 12
   ctcgagctaa cactcattcc tgttgaagc 29
<210> 13
   <211> 28
   <212> DNA
   <213> Artificial Sequence
<220>
   <223> Description of Artificial Sequence:PCR Primer
<400> 13
   ccccgggtag ggtcattcca gtctctgg 28
<210> 14
   <211> 26
   <212> DNA
   <213> Artificial Sequence
<220>
   <223> Description of Artificial Sequence:PCR Primer
<400> 14
   ctcgagtcag gcggagctca gatagc 26
<210> 15
   <211> 28
   <212> DNA
   <213> Artificial Sequence
<220>
<223> Description of Artificial Sequence:PCR Primer
<400> 15
   tctagaccat gtgtcctcag aagctaac 28
<210> 16
   <211> 25
   <212> DNA
   <213> Artificial Sequence
<220>
   <223> Description of Artificial Sequence:PCR primer
<400> 16
   ctcgagctag gatcggaccc tgcag 25
<210> 17
   <211> 29
   <212> DNA
   <213> Artificial Sequence
<220>
   <223> Description of Artificial Sequence:PCR primer
<400> 17
   ccccaagctt catactcatc aggactttc 29
<210> 18
   <211> 28
   <212> DNA
   <213> Artificial Sequence
<220>
   <223> Description of Artificial Sequence:PCR primer
<400> 18
   cccctcgagc tatttggaga aagaggtc 28
<210> 19
   <211> 49
   <212> DNA
   <213> Artificial Sequence
<220>
   <223> Description of Artificial Sequence:PCR primer
<400> 19
   ccccaagctt gtgtatctgt cagaatgtaa gccctcctgt ctctgagca 49
<210> 20
   <211> 30
   <212> DNA
   <213> Artificial Sequence
<220>
   <223> Description of Artificial Sequence:PCR primer
<400> 20
   cccctcgagc taccctcctg tctctgagca 30

## Description

### Field of the Invention

The present invention relates generally to immunoconjugates, in particular, antibody-cytokine fusion proteins useful for targeted immune therapy and general immune stimulation. More specifically, the present invention relates to the use of angiogenesis inhibitors to enhance an antibody-cytokine fusion protein mediated immune response against a preselected cell-type, for example, cells in a solid tumor.

### Background of the Invention

Antibodies have been used for treatment of human diseases for many years, primarily to provide passive immunity to viral or bacterial infection. More recently, however, antibodies and antibody conjugates have been used as anti-tumor agents. Anti-tumor activity has been difficult to demonstrate in most tumor types unless the clinical setting is one of minimal residual disease (Reithmuller *et al.,* LANCET 94: 1177-1183), or when the tumor is accessible to antibodies in the circulation, for example, in the case of B-lymphoma (Maloney *et al.* (1994) BLOOD *84:* 2457-2466). Solid tumors are much more refractory to antibody-mediated therapeutic intervention than are micrometastatic foci found in minimal residual disease settings.

Earlier studies show that the treatment of tumors with antibodies *in vivo* can be enhanced greatly by fusing immune stimulatory cytokines to an antibody molecule. However, antibody-cytokine fusion proteins were far less effective in destroying larger, solid tumors than they were for disseminated metastatic foci (Xiang *et al.* (1997) CANCER RESEARCH 57: 4948-4955, and Lode *et al.* (1998) BLOOD 91: 1706-1715).

Therefore, there still remains a need in the art for compositions and methods employing such compositions for enhancing antibody-cytokine fusion protein mediated immune responses against preselected cell-types, for example, cell-types present in solid tumors.

### Summary of the Invention

This invention is based, in part, upon the discovery that when an immunoconjugate is administered to a mammal, it is possible to create a more potent immune response against a preselected cell-type if the immunoconjugate is administered together with an angiogenesis inhibitor. In particular, it has been found that such combinations are particularly useful in mediating the immune destruction of the preselected cell-type, such as cell-types found in solid tumors and in virally-infected cells.

In one aspect, the invention provides a composition for inducing a cytocidal immune response against a preselected cell-type in a mammal. The composition comprises in combination: (i) an immunoconjugate comprising an antibody binding site capable of binding the preselected cell-type, and a cytokine capable of inducing such an immune response against the preselected cell-type in the mammal, and (ii) an angiogenesis inhibitor in an amount sufficient to enhance the immune response induced by the immunoconjugate of the combination relative to the immune response stimulated by the immunoconjugate alone.

The antibody binding site of the immunoconjugate preferably comprises, an immunoglobulin heavy chain or an antigen binding fragment thereof. The immunoglobulin heavy chain preferably comprises, in an amino-terminal to carboxy-terminal direction, an immunoglobulin variable (V_{H}) region domain capable of binding a preselected antigen, an immunoglobulin constant heavy 1 (CH1) domain, an immunoglobulin constant heavy 2 (CH2) domain, and optionally may further include an immunoglobulin constant heavy 3 (CH3) domain. In a more preferred embodiment, the immunoconjugate is a fusion protein comprising an immunoglobulin heavy chain or an antigen binding fragment thereof fused via a polypeptide bond to the cytokine. Accordingly, a preferred antibody-cytokine fusion protein comprises, in an amino-terminal to carboxy-terminal direction, (i) the antibody binding site comprising an immunoglobulin variable region capable of binding a cell surface antigen on the preselected cell-type, an immunoglobulin CH1 domain, an immunoglobulin CH2 domain (optionally a CH3 domain), and (ii) the cytokine. Methods for making and using such fusion proteins are described in detail in Gillies *et al.* (1992) PRoc. NATL. ACAD. Sci. USA 89: 1428-1432; Gillies *et al.* (1998) J. IMMUNOL. 160:6195-6203; and U.S. Patent No. 5,650,150.

The immunoglobulin constant region domains (i.e., the CH1, CH2 and/or CH3 domains) may be the constant region domains normally associated with the variable region domain in a naturally occurring antibody. Alternatively, one or more of the immunoglobulin constant region domains may derived from antibodies different from the antibody used as a source of the variable region domain. In other words, the immunoglobulin variable and constant region domains may be derived from different antibodies, for example, antibodies derived from different species. See, for example, U.S. Patent No. 4,816,567. Furthermore, the immunoglobulin variable regions may comprise framework region (FR) sequences derived from one species, for example, a human, and complementarity determining region (CDR) sequences interposed between the FRs, derived from a second, different species, for example, a mouse. Methods for making and using such chimeric immunoglobulin variable regions are disclosed, for example, in U.S. Patent Nos. 5,225,539 and 5,585,089.

The antibody-based immunoconjugates preferably further comprise an immunoglobulin light chain which preferably is covalently bonded to the immunoglobulin heavy chain by means of, for example, a disulfide bond. The variable regions of the linked immunoglobulin heavy and light chains together define a single and complete binding site for binding the preselected antigen. In other embodiments, the immunoconjugates comprise two chimeric chains, each comprising at least a portion of an immunoglobulin heavy chain fused to a cytokine. The two chimeric chains preferably are covalently linked together by, for example, one or more interchain disulfide bonds.

The invention thus provides fusion proteins in which the antigen-binding specificity and activity of an antibody is combined with the potent biological activity of a cytokine. A fusion protein of the present invention can be used to deliver the cytokine selectively to a target cell *in vivo* so that the cytokine can exert a localized biological effect in the vicinity of the target cell. In a preferred embodiment, the antibody component of the fusion protein specifically binds an antigen on a cancer cell and, as a result, the fusion protein exerts localized anti-cancer activity. In an alternative preferred embodiment, the antibody component of the fusion protein specifically binds a virus-infected cell, such as an HIV-infected cell, and, as a result, the fusion protein exerts localized anti-viral activity.

Cytokines that can be incorporated into the immunoconjugates of the invention include, for example, tumor necrosis factors, interleukins, colony stimulating factors, and lymphokines. Preferred tumor necrosis factors include, for example, tissue necrosis factor a (TNFα). Interleukins include, for example, interleukin-2 (IL-2), interleukin-4 (IL-4), interleukin-5 (IL-5), interleukin-7 (IL-7), interleukin-12 (IL-12), interleukin-15 (IL-15) and interleukin-18 (IL-18). Preferred interleukins are IL-4, IL-7, IL-2, and IL-12. Colony stimulating factors include, for example, granulocyte-macrophage colony stimulating factor (GM-CSF) and macrophage colony stimulation factor (M-CSF). Preferred lymphokines include, for example, lymphotoxin (LT). Other useful cytokines include interferons, including IFN-α, IFN-β and IFN-γ, all of which have immunological effects, as well as anti-angiogenic effects, that are independent of their anti-viral activities.

Preferred angiogenesis inhibitors useful in the practice of the invention include, for example, endostatin, angiostatin, peptides having binding affinity for α_{ν}β₃ integrin and antibodies or fragments thereof having binding affinity for ανβ3 integrin.

In a further aspect, the invention relates to the use of said compositions for inducing a cytocidal immune response against a preselected cell-type in a mammal. The use comprises administering to the mammal (i) an immunoconjugate comprising an antibody binding site capable of binding the preselected cell-type and a cytokine capable of inducing such an immune response against the preselected cell-type, and (ii) an angiogenesis inhibitor in an amount sufficient to enhance the immune response relative to the immune response stimulated by immunoconjugate alone.

In a preferred embodiment, the preselected cell-type can be a cancer cell present, for example, in a solid tumor, more preferably in a larger, solid tumor (i.e., greater than about 100 mm³). Alternatively, the preselected cell-type can be a virally-infected cell, for example, a human immunodeficiency virus (HIV) infected cell.

In another preferred embodiment, the angiogenesis inhibitor can be administered simultaneously with the immunoconjugate. Alternatively, the angiogenesis inhibitor can be administered prior to administration of the immunoconjugate. Furthermore, it is contemplated that the immunoconjugate can be administered together with a plurality of different angiogenesis inhibitors. Alternatively, it is contemplated that the angiogenesis inhibitor can be administered together with a plurality of different immunoconjugates.

Also provided are preferred dosages and administration regimes for administering the immunoconjugates in combination with the angiogenesis inhibitors.

### Brief Description of the Drawings

The foregoing and other objects, features, and advantages of the present invention, as well as the invention itself, may be more fully understood from the following description of preferred embodiments, when read together with the accompanying drawings, in which:

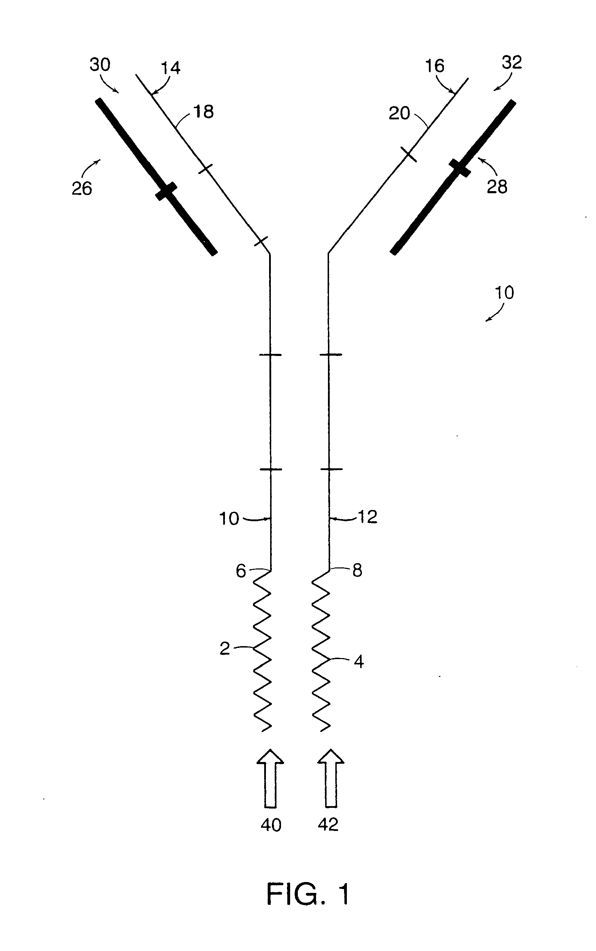

FIG. 1 is a schematic representation of an exemplary immunoconjugate useful in the practice of the invention.

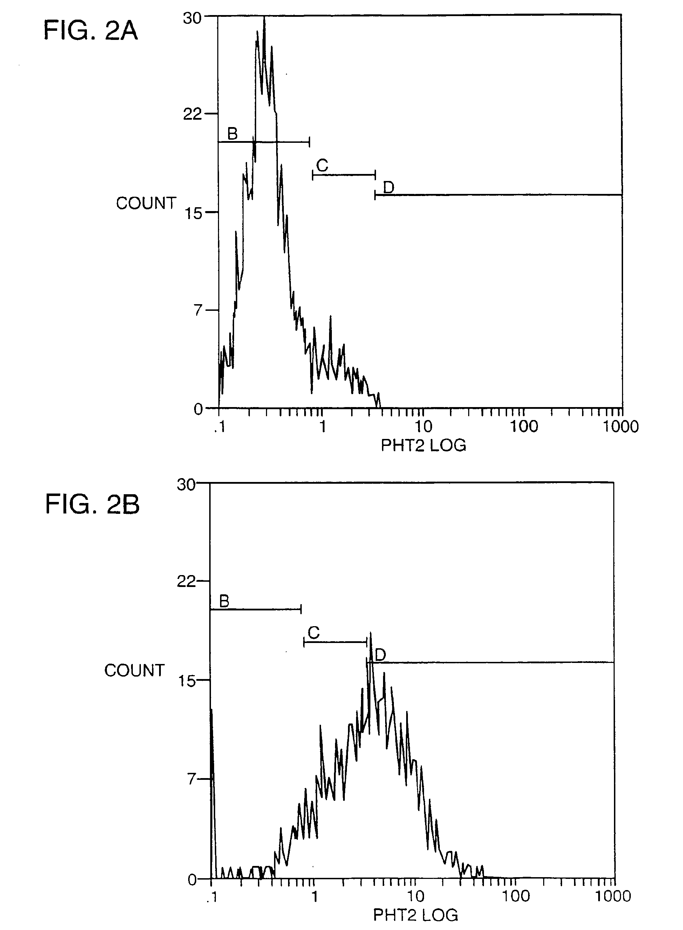

FIG. 2A and 2B are graphs depicting the expression of human EpCAM in transfected mouse Lewis lung carcinoma (LLC) cells as analyzed by fluorescence-activated cell sorting (FACS). Equal numbers of transfected cells were stained either with a secondary fluorescein isothiocyanate (FITC)-labeled anti-human Fc specific antibody alone (Panel A), or first stained with a huKS-huIL2 antibody fusion protein followed by the FITC-labeled anti-human Fc specific antibody (Panel B);

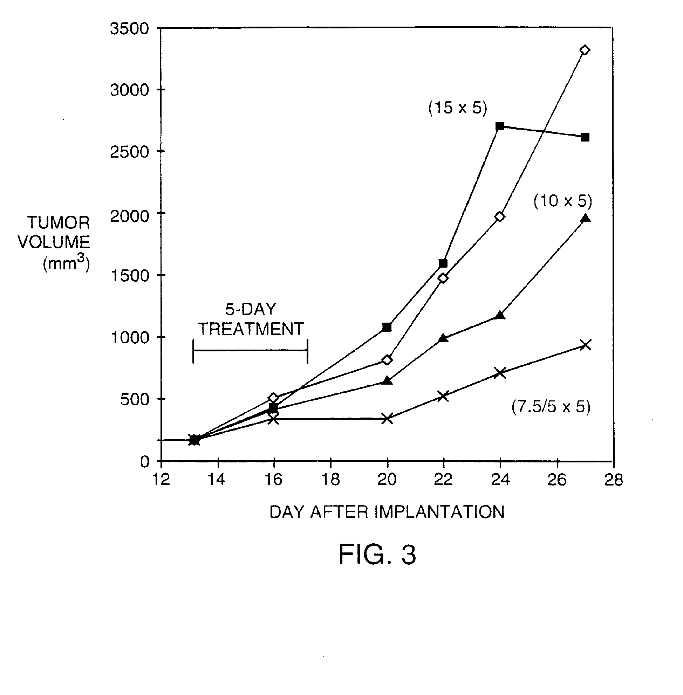

FIG. 3 is a line graph depicting the effects on subcutaneous tumors of an antibody-cytokine fusion protein administered either alone or in combination with a second antibody-cytokine fusion protein in which the cytokine has anti-angiogenic activity. Treatment for 5 days was initiated 13 days after implantation of LLC cells. The mice were treated with phosphate buffered saline (open diamonds); 15 µg/day of huKS-muγ2a-muIL2 fusion protein alone (closed squares); 10 µg/day of a huKS-muγ2a-muIL12 fusion protein (closed triangles); and a combination of 7.5 µg/day of the huKS-muγ2a-muIL2 fusion protein and 5 µg/day of the huKS-muγ2a-muIL12 fusion protein (crosses);

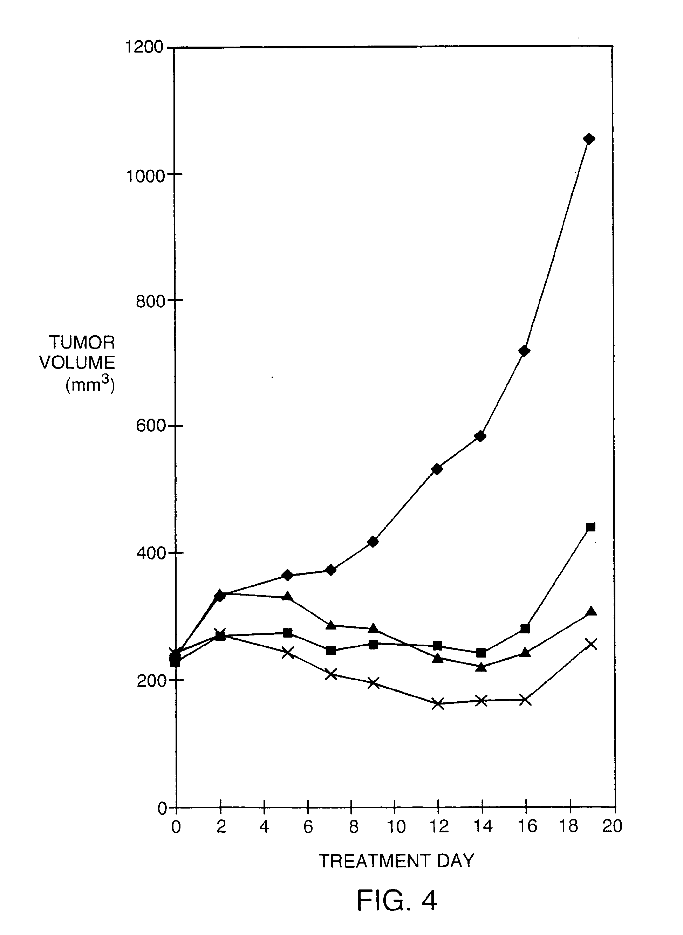

FIG. 4 is a line graph depicting the effects on subcutaneous tumors of an antibody-cytokine fusion protein administered either alone or in combination with an endostatin fusion protein. The size of CT26/EpCAM subcutaneous tumors were monitored in mice treated with phosphate buffered saline (closed diamonds), a muFc-muEndo fusion protein (closed squares), a huKS-huγ4-huIL2 fusion protein (closed diamond), and a combination of the muFc-muEndo fusion protein and the huKS-huγ4-huIL2 fusion protein (crosses); and

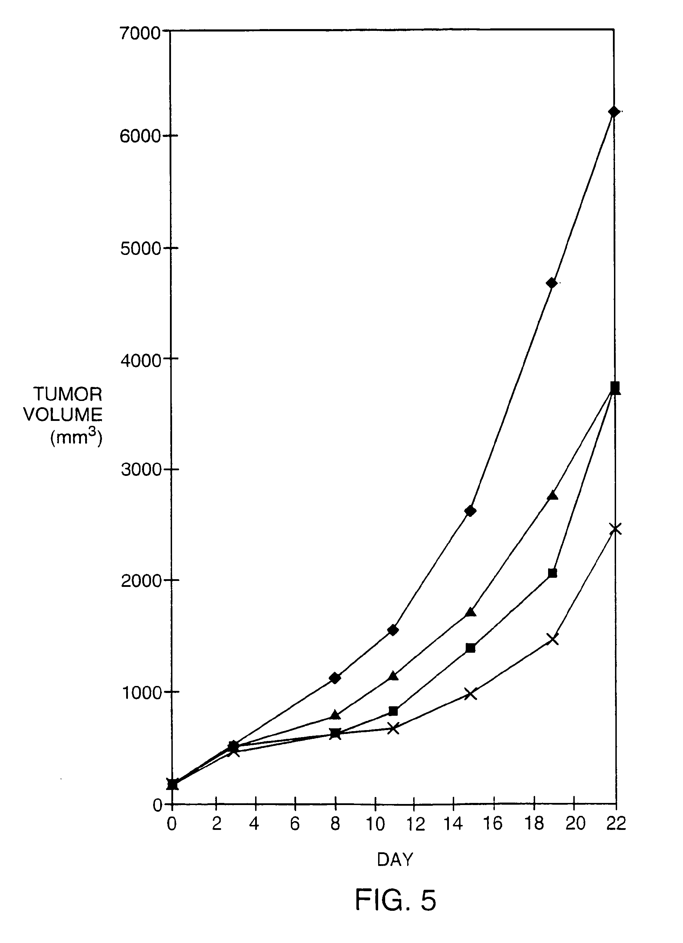

FIG. 5 is a line graph depicting the effect on subcutaneous tumors of an antibody-cytokine fusion protein administered either alone or in combination with indomethacin. The size of LLC-EpCAM subcutaneous tumors were monitored in mice treated with phosphate buffered saline (closed diamonds), a huKS-huγl-huIL2 fusion protein (closed squares), indomethocin (closed triangles), and a combination of the huKS-huγ1-huIL2 fusion protein and indomethocin (crosses).

### Detailed Description of the Invention

It has now been discovered that cytocidal immune responses initiated by an immunoconjugate against a preselected cell-type can be significantly enhanced by administering the immunoconjugate together with an angiogenesis inhibitor. The combined therapy is particularly effective in mediating the immune destruction of a diseased tissue, such as, an established tumor or virally-infected cells. The present invention describes methods for making and using useful immunoconjugates, as well as assays useful for testing their pharmacokinetic activities in pre-clinical *in vivo* animal models when they are combined with suitable angiogenesis inhibitors.

As used herein, the term "cytocidal immune response" is understood to mean any immune response in a mammal, either humoral or cellular in nature, that is stimulated by the immunoconjugate of the invention and which either kills or otherwise reduces the viability of a preselected cell-type in the mammal. The immune response may include one or more cell types, including T cells, natural killer (NK) cells and macrophages.

As used herein, the term "immunoconjugate" is understood to mean a conjugate of (i) an antibody binding site having binding specificity for, and capable of binding a surface antigen on a cancer cell or a virally-infected cell, and (ii) a cytokine that is capable of inducing or stimulating a cytocidal immune response against the cancer or virally-infected cell. Accordingly, the immunoconjugate is capable of selectively delivering the cytokine to a target cell *in vivo* so that the cytokine can mediate a localized immune response against the target cell. For example, if the antibody component of the immunoconjugate selectively binds an antigen on a cancer cell, for example, a cancer cell in a solid tumor, in particular, a larger solid tumor of greater than about 100 mm³, the immunoconjugate exerts localized anti-cancer activity. Alternatively, if the antibody component of the immunoconjugate selectively binds an antigen on a virally-infected cell, such as a human immunodeficiency virus (HIV) infected cell, the immunoconjugate exerts localized anti-viral activity.

As used herein, the term "antibody binding site" is understood to mean at least a portion of an immunoglobulin heavy chain, for example, an immunoglobulin variable region capable of binding the preselected cell-type. The antibody binding site also preferably comprises at least a portion of an immunoglobulin constant region including, for example, a CH1 domain, a CH2 domain, and optionally, a CH3 domain. Furthermore, the immunoglobulin heavy chain may be associated, either covalently or non-covalently, to an immunoglobulin light comprising, for example, an immunoglobulin light chain variable region and optionally light chain constant region. Accordingly, it is contemplated that the antibody binding site may comprise an intact antibody or a fragment thereof capable of binding the preselected cell-type.

With regard to the immunoconjugate, it is contemplated that the antibody fragment may be linked to the cytokine by a variety of ways well known to those of ordinary skill in the art. For example, the antibody binding site preferably is linked via a polypeptide bond to the cytokine in a fusion protein construct. Alternatively, the antibody binding site may be chemically coupled to the cytokine via reactive groups, for example, sulfhydryl groups, within amino acid sidechains present within the antibody binding site and the cytokine.

As used herein, the term "cytokine" is understood to mean any protein or peptide, analog or functional fragment thereof, which is capable of stimulating or inducing a cytocidal immune response against a preselected cell-type, for example, a cancer cell or a virally-infected cell, in a mammal. Accordingly, it is contemplated that a variety of cytokines can be incorporated into the immunoconjugates of the invention. Useful cytokines include, for example, tumor necrosis factors, interleukins, lymphokines, colony stimulating factors, interferons including species variants, truncated analogs thereof which are capable of stimulating or inducing such cytocidal immune responses. Useful tumor necrosis factors include, for example, TNF α. Useful lymphokines include, for example, LT. Useful colony stimulating factors include, for example, GM-CSF and M-CSF. Useful interleukins include, for example, IL-2, IL-4, IL-5, IL-7, IL-12, IL-15 and IL-18. Useful interferons, include, for example, IFN-α, IFN-β and IFN-γ.

The gene encoding a particular cytokine of interest can be cloned *de novo,* obtained from an available source, or synthesized by standard DNA synthesis from a known nucleotide sequence. For example, the DNA sequence of LT is known (see, for example, Nedwin *et al.* (1985) NUCLEIC ACIDS RES. 13:6361), as are the sequences for IL-2 (see, for example, Taniguchi *et al.* (1983) NATURE 302:305-318), GM-CSF (see, for example, Gasson *et al.* (1984) SCIENCE 266:1339-1342), and TNF α (see, for example, Nedwin *et al.* (1985) NUCLEIC ACIDS RES. 13:6361).

In a preferred embodiment, the immunoconjugates are recombinant fusion proteins produced by conventional recombinant DNA methodologies, i.e., by forming a nucleic acid construct encoding the chimeric immunoconjugate. The construction of recombinant antibody-cytokine fusion proteins has been described in the prior art. *See,* for example, Gillies *et al.* (1992) PROC. NATL. ACAD. SCI. USA *89:* 1428-1432; Gillies *et al.* (1998) J. IMMUNOL. 160:6195-6203; and U.S. Patent No 5,650,150. Preferably, a gene construct encoding the immunoconjugate of the invention includes, in 5' to 3' orientation, a DNA segment encoding an immunoglobulin heavy chain variable region domain, a DNA segment encoding an immunoglobulin heavy chain constant region, and a DNA encoding the cytokine. The fused gene is assembled in or inserted into an expression vector for transfection into an appropriate recipient cell where the fused gene is expressed. The hybrid polypeptide chain preferably is combined with an immunoglobulin light chain such that the immunoglobulin variable region of the heavy chain (V_{H}) and the immunoglobulin variable region of the light chain (V_{L}) combine to produce a single and complete site for binding a preselected antigen. In a preferred embodiment, the immunoglobulin heavy and light chains are covalently coupled, for example, by means of an interchain disulfide bond. Furthermore, two immunoglobulin heavy chains, either one or both of which are fused to a cytokine, can be covalently coupled, for example, by means of one or more interchain disulfide bonds.

Figure 1 shows a schematic representation of an exemplary immunoconjugate 10. In this embodiment, cytokine molecules 2 and 4 are peptide bonded to the carboxy termini 6 and 8 of CH3 regions 10 and 12 of antibody heavy chains 14 and 16. V_{L} regions 26 and 28 are shown paired with V_{II} regions 18 and 20 in a typical IgG configuration, thereby providing two antigen binding sites 30 and 32 at the amino terminal ends of immunoconjugate 10 and two cytokine receptor-binding sites 40 and 42 at the carboxy ends of immunoconjugate 10. Of course, in their broader aspects, the immunoconjugates need not be paired as illustrated or only one of the two immunoglobulin heavy chains need be fused to a cytokine molecule.

Immunoconjugates of the invention may be considered chimeric by virtue of two aspects of their structure. First, the immunoconjugate is chimeric in that it includes an immunoglobulin heavy chain having antigen binding specificity linked to a given cytokine. Second, an immunoconjugate of the invention may be chimeric in the sense that it includes an immunoglobulin variable region (V) and an immunoglobulin constant region (C), both of which are derived from different antibodies such that the resulting protein is a V/C chimera. For example, the variable and constant regions may be derived from naturally occurring antibody molecules isolatable from different species. See, for example, U.S. Patent 4,816,567. Also embraced are constructs in which either or both of the immunoglobulin variable regions comprise framework region (FR) sequences and complementarity determining region (CDR) sequences derived from different species. Such constructs are disclosed, for example, in Jones *et al.* (1986) NATURE 321: 522-525, Verhoyen *et al.* (1988) SCIENCE 239: 1534-1535, and U.S. Patent Nos. 5,225,539 and 5,585,089. Furthermore, it is contemplated that the variable region sequences may be derived by screening libraries, for example, phage display libraries, for variable region sequences that bind a preselected antigen with a desired affinity. Methods for making and screening phage display libraries are disclosed, for example, in Huse *et al.* (1989) SCIENCE 246: 1275-1281 and Kang *et al.* (1991) PROC. NAIL ACAD. SCI. USA 88: 11120-11123.

The immunoglobulin heavy chain constant region domains of the immunoconjugates can be selected from any of the five immunoglobulin classes referred to as IgA (Igα), IgD (Igδ), IgE (Igε), IgG (Igγ), and IgM (Igµ). However, immunoglobulin heavy chain constant regions from the IgG class are preferred. Furthermore, it is contemplated that the immunoglobulin heavy chains may be derived from any of the IgG antibody subclasses referred to in the art as IgG1, IgG2, IgG3 and IgG4. As is known, each immunoglobulin heavy chain constant region comprises four or five domains. The domains are named sequentially as follows: CH1-hinge-CH2-CH3-(-CH4). CH4 is present in IgM, which has no hinge region. The DNA sequences of the heavy chain domains have cross homology among the immunoglobulin classes, for example, the CH2 domain of IgG is homologous to the CH2 domain of IgA and IgD, and to the CH3 domain of IgM and IgE. The immunoglobulin light chains can have either a kappa (κ) or lambda (λ) constant chain. Sequences and sequence alignments of these immunoglobulin regions are well known in the art (see, for example, Kabat *et al., "Sequences of Proteins of Immunological Interest,"* U.S. Department of Health and Human Services, third edition 1983, fourth edition 1987, Huck *et al.* (1986) Nuc. ACIDS RES. 14: 1779-1789).

In preferred embodiments, the variable region is derived from an antibody specific for a preselected cell surface antigen (an antigen associated with a diseased cell such as a cancer cell or virally-infected cell), and the constant region includes CH1, and CH2 (and optionally CH3) domains from an antibody that is the same or different from the antibody that is the source of the variable region. In the practice of this invention, the antibody portion of the immunoconjugate preferably is non-immunogenic or is weakly immunogenic in the intended recipient. Accordingly, the antibody portion, as much as possible, preferably is derived from the same species as the intended recipient. For example, if the immunoconjugate is to be administered to humans, the constant region domains preferably are of human origin. See, for example, U.S. Patent No. 4,816,567. Furthermore, when the immunoglobulin variable region is derived from a species other than the intended recipient, for example, when the variable region sequences are of murine origin and the intended recipient is a human, then the variable region preferably comprises human FR sequences with murine CDR sequences interposed between the FR sequences to produce a chimeric variable region that has binding specificity for a preselected antigen but yet while minimizing immunoreactivity in the intended host. The design and synthesis of such chimeric variable regions are disclosed in Jones *et al.* (1986) NATURE 321: 522-525, Verhoyen *et al.* (1988) SCIENCE 239: 1534-1535, and U.S. Patent Nos. 5,225,539 and 5,585,089. The cloning and expression of a humanized antibody-cytokine fusion protein, KS-1/4 anti-EpCAM antibody - IL-12 fusion protein, as well as its ability to eradicate established colon carcinoma metastases has been described in Gillies *et al.* (1998) J. IMMUNOL. 160: 6195-6203.

The gene encoding the cytokine is joined, either directly or by means of a linker, for example, by means of DNA encoding a (Gly₄-Ser)₃ linker in frame to the 3' end of the gene encoding the immunoglobulin constant region (e.g., a CH2 or CH3 exon). In certain embodiments, the linker can comprise a nucleotide sequence encoding a proteolytic cleavage site. This site, when interposed between the immunoglobulin constant region and the cytokine, can be designed to provide for proteolytic release of the cytokine at the target site. For example, it is well known that plasmin and trypsin cleave after lysine and arginine residues at sites that are accessible to the proteases. Many other site-specific endoproteases and the amino acid sequences they cleave are well-known in the art. Preferred proteolytic cleavage sites and proteolytic enzymes that are reactive with such cleavage sites are disclosed in U.S. Patent Nos. 5,541,087 and 5,726,044.

The nucleic acid construct optionally can include the endogenous promoter and enhancer for the variable region-encoding gene to regulate expression of the chimeric immunoglobulin chain. For example, the variable region encoding genes can be obtained as DNA fragments comprising the leader peptide, the VJ gene (functionally rearranged variable (V) regions with joining (J) segment) for the light chain, or VDJ gene for the heavy chain, and the endogenous promoter and enhancer for these genes. Alternatively, the gene encoding the variable region can be obtained apart form endogenous regulatory elements and used in an expression vector which provides these elements.

Variable region genes can be obtained by standard DNA cloning procedures from cells that produce the desired antibody. Screening of the genomic library for a specific functionally rearranged variable region can be accomplished with the use of appropriate DNA probes such as DNA segments containing the J region DNA sequence and sequences downstream. Identification and confirmation of correct clones is achieved by sequencing the cloned genes and comparison of the sequence to the corresponding sequence of the full length, properly spliced mRNA.

The target antigen can be a cell surface antigen of a tumor or cancer cell, a virus-infected cell or another diseased cell. Genes encoding appropriate variable regions can be obtained generally from immunoglobulin-producing lymphoid cell lines, For example, hybridoma cell lines producing immunoglobulin specific for tumor associated antigens or viral antigens can be produced by standard somatic cell hybridization techniques well known in the art (see, for example. U.S. Pat. No. 4,196,265). These immunoglobulin producing cell lines provide the source of variable region genes in functionally rearranged form. The variable region genes typically will be of murine origin because this murine system lends itself to the production of a wide variety of immunoglobulins of desired specificity. Furthermore, variable region sequences may be derived by screening libraries, for example, phage display libraries, for variable region sequences that bind a preselected antigen with a desired affinity. Methods for making and screening phage display libraries are disclosed, for example, in Huse *et al.* (1989) SCIENCE 246: 1275-1281 and Kang *et al.* (1991) PROC. NATL. ACAD. SCI. USA 88: 11120-11123.

The DNA fragment encoding containing the functionally active variable region gene is linked to a DNA fragment containing the gene encoding the desired constant region (or a portion thereof). Immunoglobulin constant regions (heavy and light chain) can be obtained from antibody-producing cells by standard gene cloning techniques. Genes for the two classes of human light chains (κ and λ) and the five classes of human heavy chains (α, δ, ε, γ and µ) have been cloned, and thus, constant regions of human origin are readily available from these clones.

The fused gene encoding the hybrid immunoglobulin heavy chain is assembled or inserted into an expression vector for incorporation into a recipient cell. The introduction of the gene construct into plasmid vectors can be accomplished by standard gene splicing procedures. The chimeric immunoglobulin heavy chain an be co-expressed in the same cell with a corresponding immunoglobulin light chain so that a complete immunoglobulin can be expressed and assembled simultaneously. For this purpose, the heavy and light chain constructs can be placed in the same or separate vectors.

Recipient cell lines are generally lymphoid cells. The preferred recipient cell is a myeloma (or hybridoma). Myelomas can synthesize, assemble, and secrete immunoglobulins encoded by transfected genes and they can glycosylate proteins. Particularly preferred recipient or host cells include Sp2/0 myeloma which normally does not produce endogenous immunoglobulin, and mouse myeloma NS/0 cells. When transfected, the cell produces only immunoglobulin encoded by the transfected gene constructs. Transfected myelomas can be grown in culture or in the peritoneum of mice where secreted immunoconjugate can be recovered from ascites fluid. Other lymphoid cells such as B lymphocytes can be used as recipient cells.

There are several methods for transfecting lymphoid cells with vectors containing the nucleic acid constructs encoding the chimeric immunoglobulin chain. For example, vectors may be introduced into lymphoid cells by spheroblast fusion (see, for example, Gillies *et al.* (1989) BIOTECHNOL. 7: 798-804). Other useful methods include electroporation or calcium phosphate precipitation (see, for example, Sambrook *et al.* eds (1989) *"Molecular Cloning: A Laboratory Manual,"* Cold Spring Harbor Press).

Other useful methods of producing the immunoconjugates include the preparation of an RNA sequence encoding the construct and its translation in an appropriate *in vivo* or *in vitro* expression system. It is contemplated that the recombinant DNA methodologies for synthesizing genes encoding antibody-cytokine fusion proteins, for introducing the genes into host cells, for expressing the genes in the host, and for harvesting the resulting fusion protein are well known and thoroughly documented in the art. Specific protocols are described, for example, in Sambrook *et al.* eds (1989) *"Molecular Cloning: A Laboratory Manual,"* Cold Spring Harbor Press.

It is understood that the chemically coupled immunoconjugates may be produced using a variety of methods well known to those skilled in the art. For example, the antibody or an antibody fragment may be chemically coupled to the cytokine using chemically reactive amino acid side chains in the antibody or antibody fragment and the cytokine. The amino acid side chains may be covalently linked, for example, via disulfide bonds, or by means of homo- or hetero-bifunctional crosslinking reagents including, for example, N-succinimidyl 3 (-2-pyridyylditio)proprionate, *m*-maleimidobenzoyl-N-hydroxysuccinate ester, m-maleimidobenzoyl-N-hydroxysulfosuccinimide ester, and 1,4-di-[3'(2'-pyridylthio) propionamido] butane, all of which are available commercially from Pierce, Rockford, IL.

It is understood that the term "angiogenesis inhibitor" as used herein, refers to any molecule that reduces or inhibits the formation of new blood vessels in a mammal. With regard to cancer therapy, the angiogenesis inhibitor reduces or inhibits the formation of new blood vessels in or on a tumor, preferably in or on a solid tumor. It is contemplated that useful angiogenesis inhibitors may be identified using a variety of assays well known and used in the art. Such assays include, for example, the bovine capillary endothelial cell proliferation assay, the chick chorioallantoic membrane (CAM) assay or the mouse corneal assay. However, the CAM assay is preferred (see, for example, O'Reilly *et al.* (1994) CELL 79: 315-328 and O'Reilly *et al.* (1997) CELL 88: 277-285). Briefly, embryos with intact yolks are removed from fertilized three day old white eggs and placed in a petri dish. After incubation at 37°C, 3% CO₂ for three days, a methylcellulose disk containing the putative angiogenesis inhibitor is applied to the chorioallantoic membrane of an individual embryo. After incubation for about 48 hours, the chorioallantoic membranes were observed under a microscope for evidence of zones of inhibition.

Numerous angiogenesis inhibitors are well known and thoroughly documented in the art. Examples of angiogenesis inhibitors useful in the practice of the invention include, for example, protein/peptide inhibitors of angiogenesis such as: angiostatin, a proteolytic fragment of plasminogen (O'Reilly *et al.* (1994) CELL 79: 315-328, and U.S. Patent Nos. 5,733,876; 5,837,682; and 5,885,795); endostatin, a proteolytic fragment of collagen XVIII (O'Reilly *et al.* (1997) CELL *88*: 277-285 and U.S. Patent No. 5,854,205); peptides containing the RGD tripeptide sequence and capable of binding the αᵥβ₃ integrin (Brooks *et al.* (1994) CELL *79:* 1157-1164); and certain antibodies and antigen binding fragments thereof and peptides that interact with the αᵥβ₃ integrin found on tumor vascular epithelial cells (Brooks *et al., supra*).Endostatin and angiostatin, however, currently are most preferred.

As used herein, it is understood that an antibody portion of the immunoconjugate specifically binds a preselected antigen, a cytokine specifically binds a receptor for the cytokine, or an angiogenesis inhibitor specifically binds a receptor for the inhibitor, if the binding affinity for the antigen or receptor is greater than 10⁵ M⁻¹, and more preferably greater than 10⁷ M⁻¹. As used herein, the terms angiostatin, endostatin, TNF, IL, GM-CSF, M-CSF, LT, and IFN not only refer to intact proteins but also to bioactive fragments and/or analogs thereof. Bioactive fragments refer to portions of the intact protein that have at least 30%, more preferably at least 70%, and most preferably at least 90% of the biological activity of the intact proteins. Analogs refer to species and allelic variants of the intact protein, or amino acid replacements, insertions or deletions thereof that have at least 30%, more preferably at least 70%, and most preferably at least 90% of the biological activity of the intact protein.

Angiogenesis inhibitors may be co-administered simultaneously with the immunoconjugate, or administered separately by different routes of administration. Compositions of the present invention may be administered by any route which is compatible with the particular molecules. Thus, as appropriate, administration may be oral or parenteral, including intravenous and intraperitoneal routes of administration.

The compositions of the present invention may be provided to an animal by any suitable means, directly (e.g., locally, as by injection, implantation or topical administration to a tissue locus) or systemically (e.g., parenterally or orally). Where the composition is to be provided parenterally, such as by intravenous, subcutaneous, ophthalmic, intraperitoneal, intramuscular, buccal, rectal, vaginal, intraorbital, intracerebral, intracranial, intraspinal, intraventricular, intrathecal, intracisternal, intracapsular, intranasal or by aerosol administration, the composition preferably comprises part of an aqueous or physiologically compatible fluid suspension or solution. Thus, the carrier or vehicle is physiologically acceptable so that in addition to delivery of the desired composition to the patient, it does not otherwise adversely affect the patient's electrolyte and/or volume balance. The fluid medium for the agent thus can comprise normal physiologic saline (e.g., 9.85% aqueous NaCl, 0.15M, pH 7-7.4).

Preferred dosages of the immunoconjugate per administration are within the range of 0.1 mg/m² - 100 mg/m², more preferably, 1 mg/m² - 20 mg/m², and most preferably 2 mg/m² - 6 mg/m². Preferred dosages of the angiogenesis inhibitor will depend generally upon the type of angiogenesis inhibitor used, however, optimal dosages may be determined using routine experimentation. Administration of the immunoconjugate and/or the angiogenesis inhibitor may be by periodic bolus injections, or by continuous intravenous or intraperitoneal administration from an external reservoir (for example, from an intravenous bag) or internal (for example, from a bioerodable implant). Furthermore, it is contemplated that the immunoconjugate of the invention may also be administered to the intended recipient together with a plurality of different angiogenesis inhibitors. It is contemplated, however, that the optimal combination of immunoconjugates and angiogenesis inhibitors, modes of administration, dosages may be determined by routine experimentation well within the level of skill in the art.

A variety of methods can be employed to assess the efficacy of combined therapy using antibody-cytokine fusion proteins and angiogenesis inhibitors on immune responses. For example, the animal model described in Example 1, or other suitable animal model, can be used by a skilled artisan to test which angiogenesis inhibitors, or combinations of angiogenesis inhibitors, are most effective in acting synergistically with an immunoconjugate, for example, an antibody-cytokine fusion protein (for example, an antibody-IL2 fusion protein) to enhance the immune destruction of established tumors. The angiogenesis inhibitor, or combination of angiogenesis inhibitors, can be administered prior to, or simultaneously with, the course of immunoconjugate therapy and the effect on the tumor can be conveniently monitored by volumetric measurement. Further, as novel angiogenesis inhibitors are identified, a skilled artisan will be able to use the methods described herein to assess the potential of these novel inhibitors to enhance the anti-cancer activity of antibody-cytokine fusion proteins.

Alternatively, following therapy, tumors can be excised, sectioned and stained via standard histological methods, or via specific immuno-histological reagents in order to assess the effect of the combined therapy on immune response. For example, simple staining with hematoxolin and eosin can reveal differences in lymphocytic infiltration into the solid tumors which is indicative of a cellular immune response. Furthermore, immunostaining of sections with antibodies to specific classes of immune cells can reveal the nature of an induced response. For example, antibodies that bind to CD45 (a general leukocyte marker), CD4 and CD8 (for T cell subclass identification), and NK1.1 (a marker on NK cells) can be used to assess the type of immune response that has been mediated by the immunoconjugates of the invention.

Alternatively, the type of immune response mediated by the immunoconjugates can be assessed by conventional cell subset depletion studies described, for example, in Lode *et al.* (1998) BLOOD 91: 1706-1715. Examples of depleting antibodies include those that react with T cell markers CD4 and CD8, as well as those that bind the NK markers NK1.1 and asialo GM. Briefly, these antibodies are injected to the mammal prior to initiating antibody-cytokine treatment at fairly high doses (for example, at a dose of about 0.5 mg/mouse), and are given at weekly intervals thereafter until the completion of the experiment. This technique can identify the cell-types necessary to elicit the observed immune response in the mammal.

In another approach, the cytotoxic activity of splenocytes isolated from animals having been treated with the combination therapy can be compared with those from the other treatment groups. Splenocyte cultures are prepared by mechanical mincing of recovered, sterile spleens by standard techniques found in most immunology laboratory manuals. See, for example, Coligan *et al.* (eds) (1988) *"Current Protocols in Immunology,"* John Wiley & Sons, Inc. The resulting cells then are cultured in a suitable cell culture medium (for example, DMEM from GIBCO) containing serum, antibiotics and a low concentration of IL-2 (~10 U/mL). For example, in order to compare NK activity, 3 days of culture normally is optimal, whereas, in order to compare T cell cytotoxic activity, 5 days of culture normally is optimal. Cytotoxic activity can be measured by radioactively labeling tumor target cells (for example, LLC cells) with ⁵¹Cr for 30 min. Following removal of excess radiolabel, the labeled cells are mixed with varying concentrations of cultured spleen cells for 4 hr. At the end of the incubation, the ⁵¹Cr released from the cells is measured by a gamma counter which is then used to quantitate the extent of cell lysis induced by the immune cells. Traditional cytotoxic T lymphocyte (or CTL) activity is measured in this way.

The invention is illustrated further by the following non-limiting examples.

### Example 1. Animal Model.

A murine cancer model was developed to study the effect of combining antibody-cytokine fusion proteins and angiogenesis inhibitors in mediating effective immune responses against a tumor. The antibody-cytokine fusion proteins used in the following examples bind EpCAM, a human tumor antigen found on most epithelial derived tumors. (see, Perez and Walker (1989) J. IMMUNOL. 142: 3662-3667). In order to test the efficacy in an immuno-competent murine model, it was necessary to express the human antigen on the surface of a mouse tumor cell that is syngeneic with the mouse host. Lewis lung carcinoma (LLC) cells, a well known mouse lung cancer cell line, was selected for this purpose. As a result, the human tumor antigen, EpCAM, was expressed on the surface of LLC cells.

LLC cells were transfected with an expression plasmid containing a cDNA encoding human EpCAM antigen (recognized by the KS-1/4 antibody as described in Vurki *et al.* (1984) CANCER RES. 44:681), and driven by the cytomegalovirus (CMV) early promoter (Immunogen, Carlsbad, CA). The KS antigen (KSA or EpCAM) was cloned by PCR from cDNA prepared from the human prostate carcinoma cells, LnCAP. The forward primer had the oligonucleotide sequence 5' TCTAGAGCAGC**ATG**GCGCCCCCGC (SEQ ID NO: 1), in which the **ATG** is the translation initiation codon, and the reverse primer had the oligonucleotide sequence 5' CTCGAG**TTA**TGCATTGAGTTCCCT (SEQ ID NO: 2), where **TTA** is the anti-codon of the translation termination. The EpCAM cDNA was cloned into a retroviral vector pLNCS (Clontech, Palo Alto, CA) and transfection performed according to established protocols (Ausubel *et al.,* (eds) *"Current Protocols in Molecular Biology",* John Wiley & Sons). Briefly, the packaging cell line PA317 (ATCC CRL 9078) was transfected with pLNCX-EpCAM by calcium phosphate co-precipitation, and the conditioned medium containing the virus used to transfect LLC cells. G418 (Sigma Chemical Co.) was added to the transfected cells at 1 mg/mL to select for stable clones. Clones expressing human EpCAM antigen (LLC-Ep) were identified by immunostaining and fluorescence activated cell sorting (FACS) analysis.

As depicted in FIG. 1, LLC-Ep clones stained first with a hu-KS-IL2 antibody fusion protein (see Example 2 below) followed by a fluorescein isothiocyanate (FITC)-labeled anti-human Fc specific antibody (Jackson ImmunoResearch Laboratories, West Grove, PA), exhibited a fairly uniform level of expression of human EpCAM. The level of expression in these clones was well above the level observed with clones stained with the FITC-labeled anti-human Fc specific antibody alone.

In order to show that expression of a human cell-surface protein did not increase the immunogenicity of LLC-Ep cells, C57B1/6 mice were injected subcutaneously with varying numbers of cells. All mice were found to develop rapidly progressive tumors after injection with 5 x 10⁵ cells, with roughly the same growth kinetics observed with the parental LLC cell line. All animals became moribund and were sacrificed to avoid unnecessary suffering.

### Example 2. Preparation of Antibody-cytokine or Antibody-angiogenesis Inhibitor Fusion Proteins.

A variety of antibody-cytokine fusion proteins are discussed in the following examples. In particular, Example 3 discloses the use of humanized KS-murine γ2a-murine IL2 (huKS-muγ2a - muIL2) and humanized KS-murine γ2a -murine IL12 (huKS-muγ2a -muIL12) fusion proteins. Example 4 discloses the use of the huKS-muγ2a -muIL2 fusion protein together with murine Fc-murine angiostatin (muFc-muAngio) and murine Fc-murine endostatin (muFc - muEndo) fusion proteins. Example 5 discloses the use of a humanized KS-human γ4-human IL2 (huKS-huy4-huIL2) and muFc-muEndo fusion proteins. Finally, Example 6 discloses the use of humanized KS-human huγ1-human IL2 (huKS-huγ1-huIL2) fusion protein with indomethacin. The construction of these fusion proteins is discussed below.

### huKS-huγ1-huIL2

A gene encoding huKS-huγ1-huIL2 fusion protein was prepared and expressed essentially as described in Gillies *et al.* (1998) J. IMMUNOL. 160:6195-6203 and U.S. Patent No. 5,650,150. Briefly, humanized variable regions of the mouse KS-1/4 antibody (Varki *et al.,* (1984) CANCER RES. 44:681-687) were modeled using the methods disclosed in Jones *et al.* (1986) NATURE 321: 522-525, which involved the insertion of the CDRs of each KS 1/4 variable region into the consensus framework sequences of the human variable regions with the highest degree of homology. Molecular modeling with a Silicon Graphics Indigo work station implementing BioSym software confirmed that the shapes of the CDRs were maintained. The protein sequences then were reverse translated, and genes constructed by the ligation of overlapping oligonucleotides.

The resulting variable regions were inserted into an expression vector containing the constant regions of the human κ light chain and the human Cγl heavy chain essentially as described in Gillies *et al.* (1992) PROC. NATL. ACAD. SCI. USA 89:1428-1432, except that the metallothionein promoters and immunoglobulin heavy chain enhancers were replaced by the CMV promoter/enhancer for the expression of both chains. Fusions of the mature sequences of IL-2 to the carboxy terminus of the human heavy chains were prepared as described in Gillies *et al.* (1992) PROC. NATL. ACAD. SCI. USA 89:1428-1432, except that the 3' untranslated regions of the IL-2 gene was derived from the SV40 poly(A) region.

The IL-2 fusion protein was expressed by transfection of the resulting plasmid into NS/0 myeloma cell line with selection medium containing 0.1 µM methotrexate. Briefly, in order to obtain stably transfected clones, plasmid DNA was introduced into the mouse myeloma NS/0 cells by electroporation. NS/0 cells were grown in Dulbecco's modified Eagle's medium supplemented with 10% fetal bovine serum. About 5 x 10⁶ cells were washed once with PBS and resuspended in 0.5 mL PBS. Ten µg of linearized plasmid DNA then was incubated with the cells in a Gene Pulser Cuvette (0.4 cm electrode gap, BioRad) on ice for 10 min. Electroporation was performed using a Gene Pulser (BioRad, Hercules, CA) with settings at 0.25 V and 500 µF. Cells were allowed to recover for 10 min. on ice, after which they were resuspended in growth medium and then plated onto two 96 well plates. Stably transfected clones were selected by growth in the presence of 100 nM methotrexate (MTX), which was introduced two days post-transfection. The cells were fed every 3 days for three more times, and MTX-resistant clones appeared in 2 to 3 weeks.

Expressing clones were identified by Fc or cytokine ELISA using the appropriate antibodies (see, for example, Gillies *et al.* (1989) BIOTECHNOL. 7: 798-804). The resulting fusion protein was purified by binding, and elution from protein A Sepharose (Pharmacia), in accordance with the manufacturer's instructions.

### huKS-huγ4-huIL2

A gene encoding the huKS.huγ4.huIL2 fusion protein was constructed and expressed essentially as described in U.S.S.N. 09/256,156, filed February 24, 1999, which claims priority to U.S.S.N. 60/075,887, filed February 25, 1998.

Briefly, an Igγ4 version of the huKS-huγ1-huIL2 fusion protein, described above, was prepared by removing the immunoglobulin constant region Cγ1 gene fragment from the huKS-huγ1-huIL2 expression vector and replacing it with the corresponding sequence from the human Cγ4 gene. Sequences and sequence alignments of the human heavy chain constant regions Cγ1, Cγ2, Cy3, and Cγ4 are disclosed in Huck *et al.* (1986) Nuc. ACIDS RES. 14: 1779-1789.

The swapping of the Cγ1 and Cγ4 fragments was accomplished by digesting the original Cγ1-containing plasmid DNA with Hind III and Xho I and purifying a large 7.8 kb fragment by agarose gel electrophoresis. A second plasmid DNA containing the Cγ4 gene was digested with Hind III and Nsi I and a 1.75 kb fragment was purified. A third plasmid containing the human IL-2 cDNA and SV40 poly A site, fused to the carboxyl terminus of the human Cγ1 gene, was digested with Xho I and Nsi I and the small 470 bp fragment was purified. All three fragments were ligated together in roughly equal molar amounts and the ligation product was used to transform competent *E*. *coli.* The ligation product was used to transform competent *E. coli* and colonies were selected by growth on plates containing ampicillin. Correctly assembled recombinant plasmids were identified by restriction analyses of plasmid DNA preparations from isolated transformants and digestion with Fsp I was used to discriminate between the Cγ1 (no Fsp I) and Cγ4 (one site) gene inserts.

The final vector, containing the Cγ4-IL2 heavy chain replacement, was introduced into NS/0 mouse myeloma cells by electroporation (0.25 V and 500 µF) and transfectants were selected by growth in medium containing methotrexate (0.1 µM). Cell clones expressing high levels of the huKS-huγ4-huIL2 fusion protein were identified, expanded, and the fusion protein purified from culture supernatants using protein A Sepharose chromatography. The purity and integrity of the Cγ4 fusion protein was determined by SDS-polyacrylamide gel electrophoresis. IL-2 activity was measured in a T-cell proliferation assay (Gillis *et al.* (1978) J. IMMUNOL. 120:2027-2032) and was found to be identical to that of the γl-construct.

### huKS-muγ2a-muIL2

A gene encoding the huKS-muγ2a-muIL2 fusion protein was constructed by replacing the human antibody constant regions and human IL-2 of the huKS-huγ1-huIL2 fusion protein, as described above, with the corresponding murine sequences. Specifically, the human Cγ1-IL2 DNA was replaced with a murine Cγ2a cDNA fragment fused to a DNA encoding murine IL-2. Briefly, the VH region of the huKS was joined in frame to the murine γ2a cDNA by performing overlapping PCR using overlapping oligonucleotide primers:
(sense) 5' CC GTC TCC TCA *GCCAAA ACA ACA GCC CCA TCG GTC* (SEQ ID NO: 3);
(antisense) 5' *GG GGC TGT TGT TTT GGC* TGA GGA GAC GGT GAC TGA CG (SEQ ID NO: 4);
(sense) 5' C TTA AGC **CAG ATC CAG TTG GTG CAG** (SEQ ID NO: 5); and
(antisense) 5' CC CGG GGT CCG GGA GAA GCT CTT AGT C (SEQ ID NO: 6).

The oligonucleotides of SEQ ID NOS: 3 and 4 were designed to hybridize to the junction of the V_{H} domain of huKS and the constant region of murine γ2a cDNA (in italics). In the first round of PCR, there were two separate reactions. In one reaction, the V_{H} of huKS DNA was used as the template with the oligonucleotides of SEQ ID NOS: 4 and 5. The primer of SEQ ID NO: 5 introduced an AflII (CTTAAG) restriction site upstream of the sequence encoding the mature amino terminus of huKS V_{H} (in bold). In another reaction, murine γ2a cDNA was used as the template with the oligonucleotides SEQ ID NOS: 3 and 6. The primer of SEQ ID NO: 6 hybridized to the cDNA encoding the region around the C-terminus of γ2a and introduced a XmaI (CCCGGG) restriction site for subsequent ligation to the muIL2 cDNA. PCR products from the two reactions were mixed and subjected to a second round of PCR, using the oligonucleotides of SEQ ID NOS: 5 and 6. The resulting PCR product was cloned, and upon sequence verification, the AflII-XmaI fragment encoding the V_{H} of huKS and the murine γ2a constant region was used for ligation to the DNA encoding the signal peptide at the AflII site and the muIL2 cDNA at the XmaI site.

The murine IL2 cDNA was cloned from mRNA of murine peripheral blood mononuclear cells using the oligonucleotides set forth in SEQ ID NOS: 7 and 8, namely:
(sense) 5' GGC CCG GGT AAA **GCA CCC ACT TCA AGC TCC** (SEQ ID NO. 7); and
(antisense) 5' CCCTCGAG**TTA**TTGAGGGCTTGTTG (SEQ ID NO. 8).

The primer of SEQ ID NO: 7 adapted the muIL2 (sequence in bold) to be joined to mu γ2a at the XmaI restriction site (CCCGGG). The primer of SEQ ID NO: 8 introduced an XhoI restriction site (CTCGAG) immediately after the translation termination codon (antisense in bold).

Similarly, the variable light (V_{L}) domain of huKS was joined to the mu κ cDNA sequence by overlapping PCR. The overlapping oligonucleotides used included
(sense) 5' G GAA ATA AAA C*GG GCT GAT GCT GCA CCA ACT G* (SEQ ID NO. 9);
(antisense) *5' GC AGC ATC AGC CC*GTT TTA TTT CCA GCT TGG TCC (SEQ ID NO. 10);
(sense) 5' C TTA AGC **GAG ATC GTG CTG ACC CAG** (SEQ ID NO. 11); and
(antisense) 5' CTC GAG **CTA** ACA CTC ATT CCT GTT GAA GC (SEQ ID NO. 12).

The oligonucleotides were designed to hybridize to the junction of the V_{L} of huKS and the constant region of murine κ cDNA (in italics). In the first round of PCR, there were two separate reactions. In one reaction, the V_{L} of huKS DNA was used as template, with the oligonucleotides set forth in SEQ ID NOS. 10 and 11, which introduced an AfIII (CTTAAG) restriction site upstream of the sequence encoding the mature amino terminus of huKS V_{L} (in bold). In the other reaction, murine κ cDNA was used as template, with the oligonucleotides set forth in SEQ ID NOS. 9 and 12, which introduced an XhoI restriction site after the translation termination codon (antisense in bold).

PCR products from the two reactions were mixed and subjected to a second round of PCR using the oligonucleotide primers set forth in SEQ ID NOS. 11 and 12. The resultant PCR product was cloned, and upon sequence verification, the AfIII-XhoI fragment encoding the V_{L} of huKS and the murine κ constant region was ligated to the DNA encoding the signal peptide at the AfIII site.

Both the murine heavy and light chain sequences were used to replace the human sequences in pdHL7. The resulting antibody expression vector, containing a dhfr selectable marker gene, was electroporated (0.25V, 500µF) into murine NS/0 myeloma cells and clones selected by culturing in medium containing 0.1 µM methotrexate. Transfected clones, resistant to methotrexate, were tested for secretion of antibody determinants by standard ELISA methods. The fusion proteins were purified via protein A Sepharose chromatography according to the manufacturers instructions.

### huKS-muγ2a-muIL12

A gene encoding the huKS-muγ2a-muIL12 fusion protein was constructed and expressed essentially as described in U.S.S.N. 08/986,997, filed December 8, 1997, and Gillies *et al.* (1998) J. IMMUNOL. 160:6195-6203. Briefly, this was accomplished by fusing the murine p35 IL-12 subunit cDNA to the huKS-muγ2a heavy chain coding region prepared previously. The resulting vector then was transfected into an NS/0 myeloma cell line pre-transfected with, and capable of expressing p40 IL-12 subunit. In other words, a cell line was transfected with p40 alone and a stable, high expressing cell was selected, which was then used as a recipient for transfection by the p35 containing fusion protein (i.e., sequential transfection).

The murine p35 and p40 IL-12 subunits were isolated by PCR from mRNA prepared from spleen cells activated with Concanavalin A (5 µg/mL in culture medium for 3 days). The PCR primers used to isolate the p35 encoding nucleic acid sequence which also adapted the p35 cDNA as an XmaI-XhoI restriction fragment included:
5' CCCCGGGTAGGGTCATTCCAGTCTCTGG (SEQ ID NO: 13); and
5' CTCGAGTCAGGCGGAGCTCAGATAGC (SEQ ID NO: 14).

The PCR primer used to isolate the p40 encoding nucleic acid sequence included:
5' TCTAGACCATGTGTCCTCAGAAGCTAAC (SEQ ID NO: 15); and
5' CTCGAGCTAGGATCGGACCCTGCAG (SEQ ID NO: 16).

A plasmid vector (pdHL7-huKS-muγ2a-p35) was constructed as described (Gillies *et al.* J. IMMUNOL. METHODS 125:191) that contained a dhfr selectable marker gene, a transcription unit encoding a humanized KS antibody light chain, and a transcription unit encoding a murine heavy chain fused to the p35 subunit of mouse IL-12. The fusion was achieved by ligation of the XmaI to XhoI fragment of the adapted p35 subunit cDNA, to a unique XmaI site at the end of the CH3 exon of the murine γ2a gene prepared previously. Both the H and L chain transcription units included a cytomegalovirus (CMV) promoter (in place of the metallothionein promoter in the original reference) at the 5' end and, a polyadenylation site at the 3' end.

A similar vector (pNC-p40) was constructed for expression of the free p40 subunit which included a selectable marker gene (neomycin resistant gene) but still used the CMV promoter for transcription. The coding region in this case included the natural leader sequence of the p40 subunit for proper trafficking to the endoplasmic reticulum and assembly with the fusion protein. Plasmid pNC-p40 was electroporated into cells, and cells were plated and selected in G418-containing medium. In this case, culture supernatants from drug-resistant clones were tested by ELISA for production of p40 subunit.

The pdHL7-huKS-muγ2a-p35 expression vector was electroporated into the NS/0 cell line already expressing murine p40, as described in Gillies *et al.* (1998) J. IMMUNOL. 160: 6195-6203. Transfected clones resistant to methotrexate were tested for secretion of antibody determinants and mouse IL-12 by standard ELISA methods. The resulting protein was purified by binding to, and elution from a protein A Sepharose column in accordance with the manufacturers instructions.

### muFc-muEndo

A gene encoding the muFc-muEndo fusion protein was constructed and expressed essentially as described in U.S.S.N. 60/097,883, filed August 25, 1998.

Briefly, murine endostatin and murine Fc were expressed as a muFc-muEndostatin fusion protein. PCR was used to adapt the endostatin gene for expression in the pdCs-muFc(D₄K) vector (Lo *et al.* (1998) PROTEIN ENGINEERING 11:495-500) which contains an enterokinase recognition site Asp4-Lys (LaVallie *et al.* (1993) J. BIOL. CHEM. 268: 23311-23317). The forward primer was 5'-C CCC *AAG CTT* **CAT ACT CAT CAG GAC TTT C** (SEQ ID NO: 17), where the *AAGCTT* (*Hind*III site) was followed by sequence (in bold) encoding the N-terminus of endostatin. The reverse primer was 5'-*CCC CTC GAG* **CTA** TTT GGA GAA AGA GGT C (SEQ ID NO: 18), which was designed to put a translation STOP codon (anticodon, **CTA**) immediately after the C-terminus of endostatin, and this was followed by an *Xho*I site *(CTCGAG).*

The PCR product was cloned and sequenced, and the *Hind*III*-Xho*I fragment encoding endostatin was ligated into the pdCs-muFc(D₄K) vector. Stable NS/0 clones expressing muFc(D₄K)-muEndo were selected and assayed using an anti-muFc ELISA. The resulting fusion protein was expressed and purified via Protein A Sepharose chromatography.

### muFc-muAngio

A gene encoding the muFc-muAngio fusion protein was constructed and expressed essentially as described in U.S.S.N. 60/097,883, filed August 25, 1998.

Briefly, murine angiostatin and murine Fc were expressed as a muFc-muAngiostatin fusion protein. PCR was used to adapt the angiostatin sequence NA, kindly provided by the laboratory of Dr. Judah Folkman, Childrens Hospital, Boston, MA, for expression in the pdCs-Fc(D₄K) vector (Lo *et al.* (1998) PROTEIN ENGINEERING 11:495-500). The forward primer was 5'-C CCC *AAG CTT* **GTG TAT CTG TCA GAA TGT AAG CCC TCC TGT CTC TGA GCA (SEQ ID NO: 19),** where the *AAGCTT (Hind*III site ) was followed by sequence (in bold) encoding the N-terminus of angiostatin. The reverse primer was 5'-CCC *CTC GAG* **CTA** CCC TCC TGT CTC TGA GCA (SEQ ID NO: 20), which was designed to put a translation STOP codon (anticodon, **CTA**) immediately after the C-terminus of angiostatin, and this was followed by an *Xho*I site (*CTCGAG*).

The PCR product was cloned and sequenced, and the *Hind*III-*Xho*I fragment encoding angiostatin was ligated into the pdCs-muFc(D₄K) vector. Stable NS/0 clones expressing muFc(D₄K)-muAngio were selected and assayed using an anti-muFc ELISA. The resulting fusion protein was expressed and purified by Protein A Sepharose chromatography.

### Example 3. Combination Therapy Using KS-IL2 and KS-IL12 fusion proteins For The Treatment of LLC-Ep Tumors.

Female C57B1/6 mice were injected subcutaneously in the mid-back with LLC-Ep cells (5 x 10⁵ per mouse) grown in cell culture. After about two weeks, animals with palpable tumors in the range of 150-400 mm³ were divided into four groups, with an equal distribution of tumor sizes between the groups. The animals were treated as follows: in group 1, animals received PBS only (control group); in group 2, animals received only the huKS-muγ2a-muIL2 fusion protein; in group 3, animals received only the huKS-muγ2a-muIL12 fusion protein; and in group 4, the animals received both the huKS-muγ2a-muIL2 and the huKS-muγ2a-muIL 12 fusion proteins. Tumor growth was monitored by volumetric measurements until animals in the control group became moribund and were euthanized. Tumor volumes were measured with calipers and calculated as V = 4 π/3 (0.5L x 0.5W x 0.5H), where L is the length, W is the width, and H is the height of the tumor.

The results are summarized in FIG. 3. Mice treated with PBS are represented by open diamonds, mice treated with 15 µg/day of huKS-muγ2a-muIL2 fusion protein are represented by closed squares, mice treated with 10 µg/day of a huKS-muγ2a-muIL12 fusion protein are represented by closed triangles, and mice treated with a combination of 7.5 µg/day of the huKS-muγ2a-muIL2 fusion protein and 5 µg/day of the huKS-muγ2a-muIL12 fusion protein are represented by crosses.

As illustrated in FIG. 3, treatment with the huKS-muγ2a-muIL2 fusion protein (15 µg for 5 consecutive days) did not delay or reduce the growth of LLC-Ep tumors (closed squares). Only a slight anti-tumor effect was seen in mice treated with the huKS-muγ2a-muIL12 fusion protein alone at 10 µg per dose for five consecutive days (closed triangles). However, when the two fusion proteins were combined using half of the original amounts for each (7.5 µg of huKS-muγ2a-muIL2 and 5 µg of huKS-muγ2a-muIL12, respectively), a striking growth delay was observed (crosses) suggesting a synergistic effect between the two fusion proteins.

### Example 4. Therapy Using An Antibody-cytokine Fusion Protein And A Combination Of Angiostatin And Endostatin.

Female C57B1/6 mice can be injected subcutaneously in the mid-back with LLC-Ep cells (5 x 10⁵ per mouse) grown in cell culture. After about two weeks, animals with palpable tumors in the range of 150-400 mm³ are divided into four groups and treated as follows: (1) animals receiving only PBS; (2) animals receiving a mixture of muFc-Angio and muFc-Endo; (3) animals receiving huKS-muγ2a-muIL2 fusion protein; and (4) animals receiving both the huKS-muγ2a-muIL2 fusion protein and the mixture of muFc-Angio and muFc-Endo. The animals are injected with their respective treatments for five consecutive days.

muFc-Angio and muFc-Endo are administered to tumor-bearing mice at a dose of 5 mg/kg. Animals receiving the huKS-muγ2a-muIL2 fusion protein are treated with daily doses of 10 µg of huKS-muγ2a-muIL2 for a total of 5 days. The growth of the tumors is monitored by volumetric measurements.

It is contemplated that treatment with the mixture of muFc-Angio and muFc-Endo alone will be effective at shrinking LLC tumors over a treatment period of two weeks. It is contemplated, however, that this treatment will not be as effective over a short period of, for example, five days. It is also contemplated that animals treated with the muKS-muIL2 fusion protein alone will exhibit minimal anti-tumor activity. It is contemplated, however, that the group receiving both the huKS-muγ2a-muIL2 fusion protein and a mixture of muFc-Angio and muFc-Endo will show significantly reduced tumor growth compared to either the muFc-Angio and muFc-Endo group, or the huKS-muγ2a-muIL2 fusion protein group.

### Example 5 Combination Therapy Of Antibody-cytokine Fusion Protein And Endostatin.

Mouse CT26 carcinoma cells expressing human EpCAM were injected subcutaneously in the shaved backs of BALB/c mice (2 x 10⁶ cells per injection). When the tumors reached 100-200 mm³ in size (about 7 to 14 days), the mice were randomized into four groups, 4 mice per group. Group 1 received intravenous injections of 0.2 mL of PBS daily. Group 2 received intravenous injections of muFc-muEndostatin (320 µg/mouse) in PBS daily. Group 3 received intravenous injections of huKS-huγ4-huIL2 fusion protein (10 µg/mouse) in PBS daily for 5 days only. Group 4 received intravenous injections of a combination of huKS-huγ4-huIL2 (10 µg/mouse) and muFc-muEndo (320 µg/mouse) in PBS daily for 5 days, and thereafter daily injections of muFc-muEndo (320 µg/mouse) in PBS. Tumor volumes were measured as described in Example 3.

The results are summarized in FIG. 4. Mice treated with PBS are represented by closed diamonds, mice treated with the muFc-muEndo fusion protein are represented by closed squares, mice treated with a huKS-huγ4-huIL2 fusion protein are represented by closed diamonds, and mice treated with a combination of the muFc-muEndo fusion protein and the huKS-huγ4-huIL2 fusion protein are represented by crosses.

FIG. 4 shows that the combination of the antibody-cytokine fusion protein and the anti-angiogenic protein muFc-muEndo was superior to either agent by itself. After treatment for 19 days, the T/C ratio (average size of tumors in the treatment group/average size of tumors in the control group) for the combination therapy of huKS-huγ4-huIL2 and muFc-muEndo was 0.25, which was a significant improvement over the T/C of 0.31 for huKS-huγ4-huIL2 and 0.42 for muFc-muEndo.

### Example 6 Combination Therapy of Antibody-cytokine Fusion Protein and indomethacin.

Female C57B1/6 mice were injected subcutaneously in the mid-back with LLC-Ep cells (2 x 10⁶ cells per injection). When the tumors reached 600-1200 mm³, the mice were sacrificed. The skin overlying the tumor was cleaned with betadine and ethanol, the tumors excised and necrotic tissue discarded. A suspension of tumor cells in phosphate buffered saline was prepared by passing viable tumor tissue through a sieve and then through a series of sequentially smaller hypothermic needles of 22- to 30- gauge. The cells were adjusted to a concentration of 1 x 10⁷ cells/mL and placed on ice. C57BL/6 mice then were injected with 0.1 mL of the freshly resuspended cells (1 x 10⁶ cells/mouse) in the proximal midline of the subcutaneous dorsa.

When the tumors reached 100-200 mm³ in size (about 7 to 14 days), the mice were randomized into four groups, 5 mice per group. Group 1 received intravenous injections of 0.2 mL of PBS daily. Group 2 received 5 daily intravenous injections of huKS-huγ1-huIL2 (25 µg/mouse) in PBS. Group 3 received indomethacin orally in drinking water (20 µg/mL, or about 60-70 µg of indomethacin consumed daily per mouse) throughout the treatment period. Group 4 received 5 daily intravenous injections of huKS-huγ1-huIL2(25 µg/mouse), and indomethacin orally in drinking water (20 µg/mL) throughout the treatment period. Tumor volumes were measured as described in Example 3.

The results are presented in FIG. 5. Mice treated with PBS are represented by closed diamonds, mice treated with the huKS-huγl-huIL2 fusion protein are represented by closed squares, mice treated with indomethocin are represented by closed triangles, and mice treated with a combination of the huKS-huγ1-huIL2 fusion protein and indomethocin are represented by crosses.

FIG. 5 shows that the combination of an antibody-cytokine fusion protein and the anti-angiogenic chemical compound indomethacin was superior to either agent by itself. After treatment for 22 days, the T/C ratio for the combination therapy of huKS-huγ4-huIL2 and indomethacin was 0.40, which was a significant improvement over the T/C of 0.61 for huKS-huγ4-huIL2 and 0.60 for indomethacin.

## Claims

1. A composition comprising in combination:
(i) a fusion protein of (a) an immunoglobulin molecule comprising in amino-terminal to carboxy-terminal direction an immunoglobulin variable heavy chain domain (VH) capable of binding the antigen, an immunoglobulin constant heavy chain 1 domain (CH1) and an immunoglobulin constant heavy chain 2 domain (CH2), and (b) a cytokine fused to the carboxy-terminal of the immunoglobulin domain capable of inducing an immune response against said antigen in said mammal, wherein the fusion protein induces an immune response against an antigen of a pre-selected cell type in a mammal, and
(ii) an angiogenesis inhibitor selected from the group consisting of
(a) endostatin,
(b) angiostatin,
(c) a peptide having binding affinity for avβ3 integrin, and
(d) an antibody having binding affinity for avβ3 integrin,
in an amount, wherein the angiogenesis inhibitor enhances said immune response induced by the fusion protein relative to the fusion protein alone.

2. A composition according to claim 1, wherein the immunoglobulin domain further comprises a an immunoglobulin constant heavy chain 3 domain (CH3), interposed between the CH2 domain and the cytokine.

3. A composition of claim 1 or 2, wherein the immunoglobulin molecule further comprises an immunoglobulin light chain (VL) combined with the VH domain to produce a single and complete site for binding the antigen.

4. A composition according to any of the claims 1 to 3, wherein the cytokine of the fusion protein is selected from the group consisting of tumor necrosis factor, an interleukin, a colony stimulating factor, and a lymphokine.

5. A composition of claim 4, wherein the interleukin selected from the group IL-2, IL-4, IL-7 and IL-12.

6. A composition according to any of the claims 1 to 5, wherein the pre-selected cell type is a cancer cell.

7. The use of a composition as defined in any of the claims 1 to 5 for the manufacture of a medicament which enhances in a mammal the cytocidal immune response against a preselected cell type.

8. The use of claim 7, wherein the medicament is suitable for the treatment of cancer.

## Patentansprüche

1. Zusammensetzung, enthaltend in Kombination:
(i) ein Fusionsprotein aus (a) einem Immunglobulinmolekül, das von aminoterminaler zu carboxyterminaler Richtung eine variable Domäne einer schweren Immunglobulinkette (VH), die das Antigen binden kann, eine konstante Domäne 1 einer schweren Immunglobulinkette (CH1) und eine konstante Domäne 2 einer schweren Immunglobulinkette (CH2) umfasst, und (b) einem Cytokin, das an den Carboxyterminus der Immunglobulindomäne fusioniert ist, die eine Immunantwort gegen das Antigen in dem Säuger induzieren kann, wobei das Fusionsprotein eine Immunantwort gegen ein Antigen eines zuvor ausgewählten Zelltyps in einem Säuger induziert, und
(ii) einen Angiogeneseinhibitor, der aus der Gruppe bestehend aus
(a) Endostatin
(b) Angiostatin
(c) einem Peptid mit Bindungsaffinität zu avβ3-Integrin und
(d) einem Antikörper mit Bindungsaffinität zu avβ3-Integrin,
ausgewählt ist, in einer Menge, in der der Angiogeneseinhibitor die durch das Fusionsprotein induzierte Immunantwort verglichen mit nur dem Fusionsprotein verstärkt.

2. Zusammensetzung nach Anspruch 1, wobei die Immunglobulindomäne ferner eine konstante Domäne 3 einer schweren Immunglobulinkette (CH3) zwischen der CH2-Domäne und dem Cytokin enthält.

3. Zusammensetzung nach Anspruch 1 oder 2, wobei das Immunglobulinmolekül ferner eine leichte Immunglobulinkette (VL) enthält, die sich mit der VH-Domäne unter Bildung einer einzelnen und vollständigen Stelle zum Binden des Antigens vereinigt.

4. Zusammensetzung nach einem der Ansprüche 1 bis 3, wobei das Cytokin des Fusionsproteins aus der Gruppe bestehend aus Tumornekrosefaktor, einem Interleukin, einem koloniestimulierenden Faktor und einem Lymphokin ausgewählt ist.

5. Zusammensetzung nach Anspruch 4, wobei das Interleukin aus der Gruppe IL-2, IL-4, IL-7 und IL-12 ausgewählt ist.

6. Zusammensetzung nach einem der Ansprüche 1 bis 5, wobei der zuvor ausgewählte Zelltyp eine Krebszelle ist.

7. Verwendung einer Zusammensetzung nach einem der Ansprüche 1 bis 5 zur Herstellung eines Arzneimittels, das in einem Säuger die zytozide Immunantwort gegen einen zuvor ausgewählten Zelltyp verstärkt.

8. Verwendung nach Anspruch 7, wobei sich das Arzneimittel zur Behandlung von Krebs eignet.

## Revendications

1. Composition comprenant en combinaison :
(i) une protéine hybride constituée par (a) une molécule d'immunoglobuline qui comprend, au niveau d'une terminaison amino suivant une direction de terminaison carboxy, un domaine en chaîne lourde variable (VH) d'immunoglobuline susceptible de lier l'antigène, un domaine en chaîne lourde constante 1 (CH1) d'immunoglobuline et un domaine en chaîne lourde constante 2 (CH2) d'immunoglobuline, et par (b) une cytokine qui est fusionnée sur la terminaison carboxy du domaine d'immunoglobuline susceptible d'induire une réponse immunitaire vis-à-vis dudit antigène dans ledit mammifère, dans laquelle la protéine hybride induit une réponse immunitaire vis-à-vis d'un antigène d'un type de cellule présélectionnée dans un mammifère ; et
(ii) un inhibiteur d'angiogénèse qui est choisi parmi le groupe comprenant :
(a) endostatine ;
(b) angiostatine ;
(c) un peptide présentant une affinité de liaison pour l'intégrine avβ3 ; et
(d) un anticorps présentant une affinité de liaison pour l'intégrine avβ3,
selon une quantité selon laquelle l'inhibiteur d'angiogénèse améliore ladite réponse immunitaire qui est induite par la protéine hybride vis-à-vis de la protéine hybride seule.

2. Composition selon la revendication 1, dans laquelle le domaine d'immunoglobuline comprend en outre un domaine en chaîne lourde constante 3 (CH3) d'immunoglobuline, qui est interposé entre le domaine CH2 et la cytokine.

3. Composition selon la revendication 1 ou 2, dans laquelle la molécule d'immunoglobuline comprend en outre une chaîne légère (VL) d'immunoglobuline qui est combinée avec le domaine VH afin de produire un site unique et complet pour lier l'antigène.

4. Composition selon l'une quelconque des revendications 1 à 3, dans laquelle la cytokine de la protéine hybride est choisie parmi le groupe qui comprend un facteur de nécrose de tumeur, une interleukine, un facteur de stimulation de colonie et une lymphokine.

5. Composition selon la revendication 4, dans laquelle l'interleukine est choisie parmi le groupe IL-2, IL-4, IL-7 et IL-12.

6. Composition selon l'une quelconque des revendications 1 à 5, dans laquelle le type de cellule présélectionnée est une cellule cancéreuse.

7. Utilisation d'une composition telle que définie selon l'une quelconque des revendications 1 à 5 pour la fabrication d'un médicament qui améliore dans un mammifère la réponse immunitaire cytodidale vis-à-vis d'un type de cellule présélectionnée.

8. Utilisation selon la revendication 7, dans laquelle le médicament convient pour le traitement du cancer.
